Europäisches Patentamt

(19) European Patent Office

Office européen des brevets

(11) Publication number: **0 098 106**

**A2**

(12)

# EUROPEAN PATENT APPLICATION

(21) Application number: **83303632.0**

(22) Date of filing: **23.06.83**

(51) Int. Cl.³: **G 01 N 25/04**
**G 01 N 33/28, G 01 N 11/12**

(30) Priority: **30.06.82 GB 8218906**

(43) Date of publication of application:
**11.01.84 Bulletin 84/2**

(84) Designated Contracting States:
**DE FR GB IT NL**

(71) Applicant: **FRANK & OCKRENT LIMITED**
**7 Berners Mews**
**London W1P 3DG(GB)**

(72) Inventor: **Frank, Peter**
**18 Credition Road**
**London NW10(GB)**

(72) Inventor: **Lawrence, Thomas Frederick William**
**24 Rowney Gardens**
**Sawbridgeworth Hertfordshire CM21 0AT(GB)**

(74) Representative: **Wright, Peter David John et al,**
**R.G.C. Jenkins & Co. 12-15, Fetter Lane**
**London EC4A 1PL(GB)**

(54) **Method and apparatus for measuring characteristics of liquids.**

(57) The pour point of an oil, for example, is detected by reciprocating an actuator which first allows a member to sink under its own weight into the liquid, and then lifts the member back to its original position. The temperature at which the member fails to sink by a predetermined distance within a predetermined time period is noted. The member is made of heat insulating plastics material. Means are provided to produce an audible, visual and/or electrical signal when the member has shifted downwardly by the predetermined distance.

EP 0 098 106 A2

./...

Croydon Printing Company Ltd.

FIG.1

- 1 -

METHOD AND APPARATUS FOR MEASURING CHARACTERISTICS OF LIQUIDS

This invention relates to a method and an apparatus for measuring characteristics of liquids, and particularly the temperatures at which liquids cease to flow. The invention is particularly, but not exclusively, applicable to the measurement of the pour point of an oil.

One of the more important tests carried out on a petroleum oil is the measurement of its pour point. For many years, this has been done by progressively cooling a test jar containing the oil, and repeatedly removing the test jar and tilting it to see whether the oil flows. Details of this method are described in the IP Standard Designation IP 15/67 (Reapproved 1975). This corresponds to the American Standard Designation ASTM D97-66 (Reapproved 1971).

This method has a number of disadvantages. It is very difficult, particularly with some oils which "creep", to determine whether or not the oil is actually flowing, albeit slowly. The test jar may frost up, which would make this even more difficult. The test is somewhat subjective, and requires operators with skill and experience to judge accurately whether or not flowing of the oil is occurring.

The removal of the test jar from its container tends to warm and disturb the oil, which results in inaccuracy. Also, the operation is very tedious, which can lead to errors.

The above-mentioned standard requires that duplicate results by the same operator should not differ by more than $3^{\circ}C$, and that results from two different laboratories should not differ by more than $6^{\circ}C$. The results are thus permitted to vary within relatively wide ranges, but even these requirements can be difficult to achieve.

Furthermore, the test is very slow, and 3 to 4 hours may be required for a single batch of tests to be carried out.

An attempt has been made to overcome these problems by providing an automatic machine, which has a paddle submerged in the oil and rotatable by a limited torque motor. As the temperature decreases, the motor attempts to rotate the paddle every $3^{\circ}C$. When it fails to do this, the pour point has been reached.

This arrangement is very expensive, and very unreliable. It can only handle one test at a time,

0098106

- 3 -

and therefore a batch of tests would take a long time to be carried out.

The inventors have considered a system in which the pour point is instead measured by supporting a member submerged in the oil on a threaded collar. As the temperature drops, the collar is screwed down in successive operations, and each time it is observed whether the submerged member sinks. If the member does not sink such that it is again supported by the collar within a predetermined time period, then the pour point has been reached.

However, tests indicate that this proposal does not adequately solve the problems mentioned above.

British Patent No. 1,522,275 describes a device for comparing the rate of flow of a liquid petroleum product at different temperatures. The device includes a core immersed in the product and secured to a piston which is caused to move within a tube under the action of a given force, e.g. produced by suction. The time taken for the core to rise a rather substantial predetermined distance is measured at different temperatures of the liquid. This is a rather expensive arrangement and requires complicated structures for controllably applying the predetermined force to the piston. Furthermore, the device would not be capable of producing consistently accurate results.

According to the present invention there is provided a device for establishing the temperature at which a liquid ceases to flow, the device comprising a member movable from a first fixed position under a predetermined force in, or into, the liquid, and indicating means for indicating when the member has reached a second fixed position spaced a predetermined distance from said first fixed position, characterised by a reciprocating actuator which has an initial position in which it supports said member in the member's first fixed position and which is movable away from the initial position to permit the member to move under said force away from the member's first position, the actuator thereafter being movable back to its initial position during the course of which it brings the member back to the member's first position.

The invention also extends to a method for establishing the temperature at which a liquid ceases to flow, the method using a device as set out in the preceding paragraph.

The use of a reciprocating actuator enables the device to be much simpler both in terms of structure and method of operation than the device of British Patent No. 1,522,275. All that is needed to operate the device is a simple reciprocation of the actuator, which is repeated at progressively lower temperatures. Each time the actuator is moved away from its initial position, it is determined using the indicating means whether the member moves between its first and second fixed positions within a predetermined time period, and if not the actuator is returned to its initial position, which automatically returns the member to its first fixed position, the temperature decreased and the operation repeated. The temperature of the liquid at which the member fails to reach its second fixed position within the predetermined time period is noted.

The member preferably moves under its own weight; this further simplifies the structure of the device.

The actuator is preferably movable between its initial position and a second position, and is preferably biased to one of these positions. In the preferred embodiment, the biasing means is arranged to retract the actuator to its initial position, during the course of which the actuator shifts the member back to the member's first position.

It is desirable for the predetermined distance between the first and second fixed positions of the member to be small, so that the member reciprocates in a relatively small region of the liquid. The temperature in this region can be kept substantially uniform, and measured accurately. Accordingly, accurate results can be achieved as compared with the device of British Patent No. 1,522,275, in which the core moves through a large region of the liquid, which would have a substantial temperature gradient. The results are further improved in the preferred embodiment of the invention in which the entire path of movement of the member is in proximity to the temperature-sensitive portion of a thermometer. Preferably, the member is a ring which surrounds the temperature-sensitive portion throughout the movement of the member.

It is preferred that the distance between the first and second positions of the member be not more than 1 cm, a particularly preferred distance being 1 mm, to ensure that the member does not leave the region of the liquid in which the temperature is accurately sensed.

Experiments have shown that such a device can give very accurate results compared with the other arrangements described above.

By arranging for the member to reciprocate through a small distance, the results achieved by the device can be made more consistent with the results achieved using the standard referred to above, because the testing can be confined to a region of the liquid which is adjacent the surface, where the characteristics may differ from those within the bulk of the liquid. In addition, the time taken for testing is substantially reduced as compared with, for example, the arrangement of British Patent No. 1,522,275. By way of example, . the predetermined time limit against which the movement of the member between its first and second positions is compared may be 10 seconds or less, a preferred figure being 7 seconds.

The indicating means may be simply a pair of markings, one of which is carried by the member, which meet when the member reaches its second position. Preferably however, the indicating means is an actuable device which is operated by the member reaching its second position. Means may be provided to produce a signal indicating that the pour point has been reached in response to the indicating means failing to indicate that the predetermined amount of movement has taken place within the predetermined time limit.

For example, the indicating means may comprise an optical sensor which is responsive to the interruption of an optical path by the member or a structure attached thereto. The term "optical" is used in a general sense, and is intended to refer to any kind of electromagnetic radiation, including for example infrared radiation, rather than merely visible light.

The device may alternatively, or additionally, have an indication means which has a first part comprising a magnet, and a second part comprising an indicator member which is movable by the magnet. One of the parts is attached to the member, and the other part is either located in a fixed position, or preferably carried by the reciprocating actuator. Such a device is particularly advantageous for manual operation.

In a preferred embodiment, the member moves in a first direction (normally downwardly) during the testing procedure, but the reciprocating actuator is moved in a transverse direction to permit such movement of the member. The reciprocating member may for example be rotatable about an upright axis.

This arrangement has a number of advantages, particularly when using the magnet-type detection means referred to above with one part of the detection means fixed with respect to the member and the other part located on the reciprocating actuator, because one can then arrange for there to be a large separation (both laterally and vertically) between the two parts when the device is in its initial condition. This ensures that, after the indicating member has been shifted by the magnet during a checking operation, it is completely released from the force of the magnet when the actuator returns to its initial position.

Preferably, the member is allowed to move by the required amount by a simple, short movement of the actuator, e.g. a partial turn, preferably of substantially less than 360°. This simplifies both manual and automatic operation of the device.

Preferably, the member and/or a supporting structure therefor is made of a heat insulating material. The material may for example be a plastics material, e.g. polytetrafluoroethylene.

Such an arrangement avoids heating of the liquid by conduction via the member and its supporting structure. It has been found that by using heat insulating material the test can be carried out much more quickly, particularly if both the member and its supporting structure, and preferably the rest of the device, are made of heat insulating material. The substantial differences in the times required to carry out the tests suggest that heating of the liquid when using a conductive member and supporting structure resulted in a substantial reduction in the rate at which the liquid was cooled.

The use of a heat insulation material rather than, for example, metal also avoids the problem of the various parts of the device sticking together due to the low temperatures encountered in use of the device.

An arrangement embodying the invention will now be described by way of example with reference to the accompanying drawings, in which:-

Figures 1 and 2 are side elevations of a device in accordance with the invention for measuring the pour point of oils, Figure 1 showing the device in the position adopted before it is used to check whether the pour point has been reached, and Figure 2 showing the device during the checking operation;

Figures 3 and 4 are plan views of the device as shown in Figures 1 and 2, respectively; and

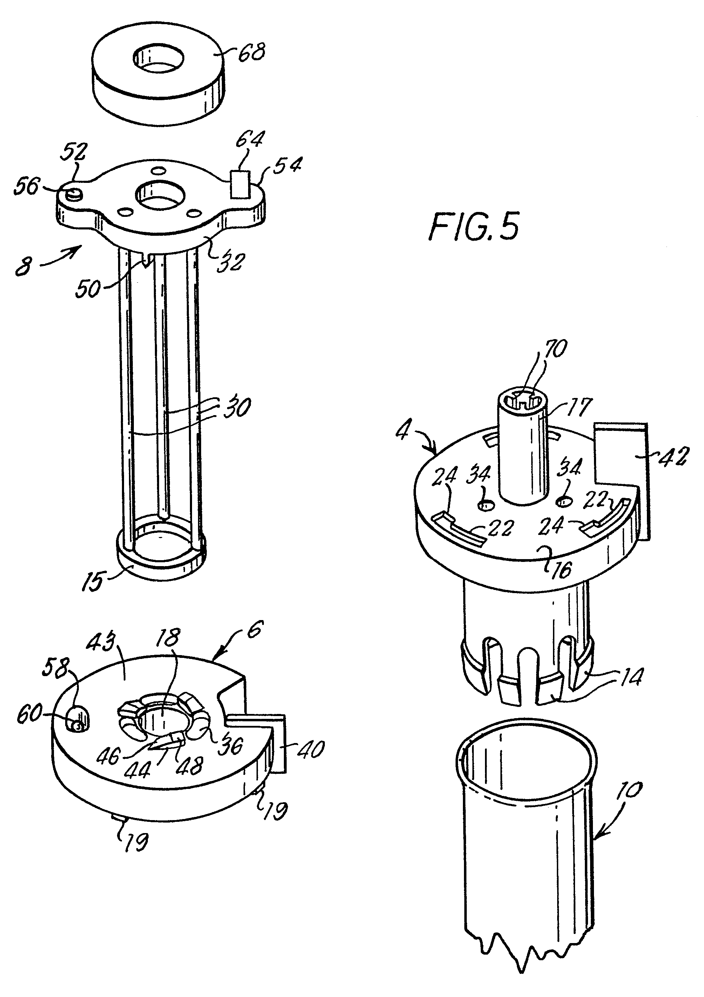

Figure 5 is an exploded view showing the various parts of the device.

Referring to the drawings, the device 2 comprises three main parts: a base section 4, a rotatable reciprocating actuator 6 and a test structure 8. The parts 4, 6 and 8 are each made of a heat insulating plastics material sold under the trade name Delrin. The three parts are assembled together, and the lower part of the device 2 is inserted into a vessel or test jar 10.

The base section 4 has a lower, cylindrical structure 12, the lower end of which is formed with cut-out portions to produce resilient legs 14 which grip the inner wall of the vessel 10 to hold the device 2 in position.

The test structure 8 is movable vertically with respect to the rest of the device 2, and has at its lower end a ring 15 which is normally submerged in oil in the vessel 10.

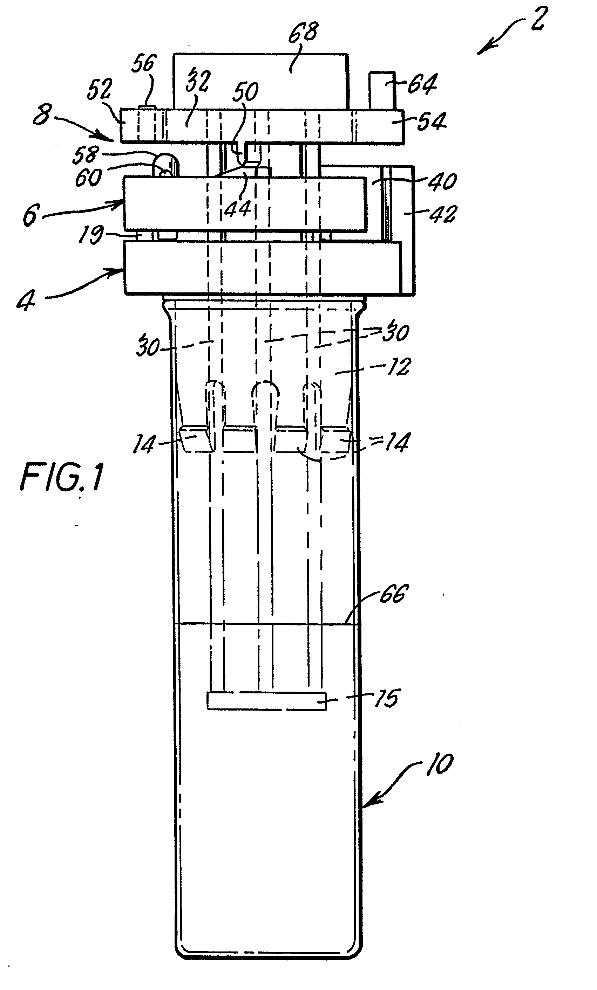

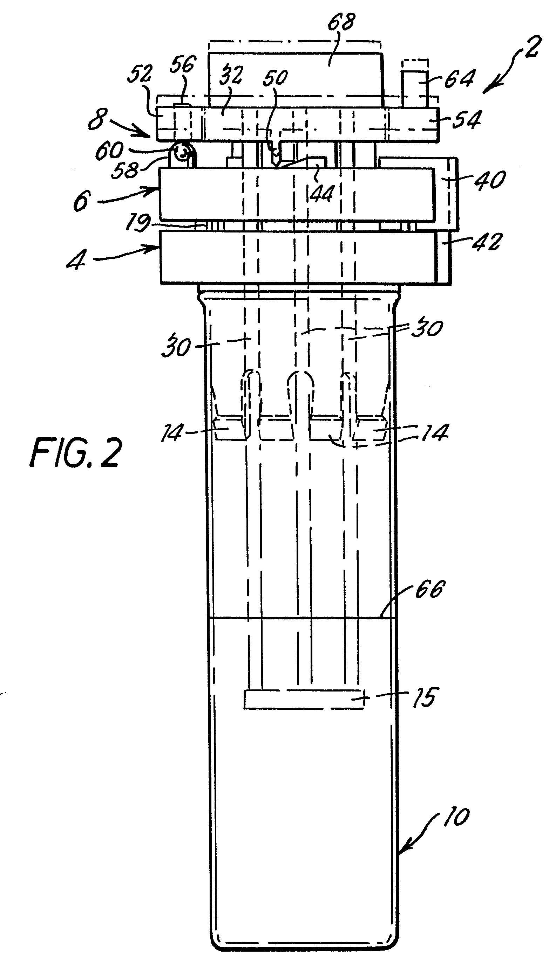

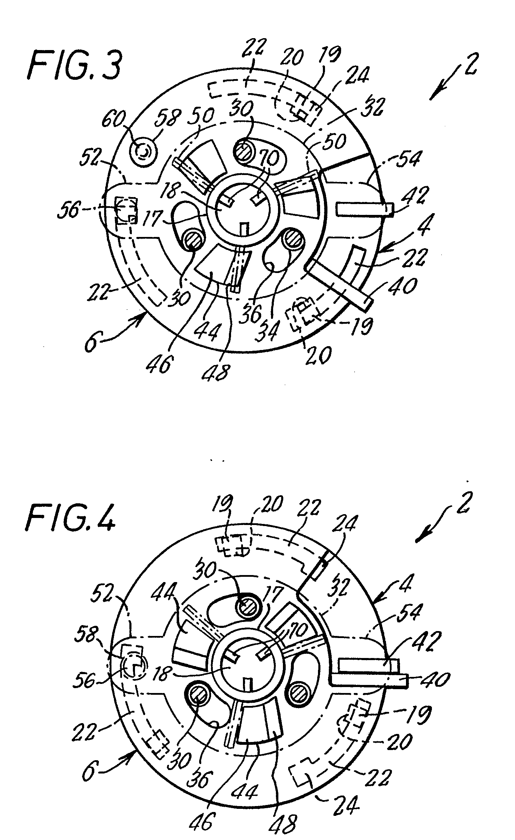

As will be described, the test structure 8 is normally supported by the actuator 6 in a relatively elevated position, as shown in Figures 1 and 3. The actuator 6 can then be operated to release the test structure 8. Assuming that an oil under test in the vessel 10 is at a temperature above its pour point, the ring 15 will then sink under the weight of the test structure 8, so that the entire test structure 8 drops to a lower position in which it is again supported on the actuator 6 (see Figures 2 and 4). If the test structure 8 does not drop, then the pour point has been reached. Accordingly, the pour point is established by noting the temperature at which the test structure 8 fails to drop within a predetermined time after operation of the actuator 6. The actual pour point may be calculated to be a predetermined offset (say +3°C) from this temperature.

If the pour point has not yet been reached, the actuator 6 is operable to raise the test structure 8 back to its elevated position. The temperature of the oil is then reduced, e.g. by 3°C, and the operation is repeated.

The detailed construction of the device 2 will now be described.

The actuator 6 is positioned on top of a support surface 16 of the base section 4 with an upright, cylindrical spigot 17 of the base section extending through a central, circular aperture 18 in the actuator 6. The actuator 6 is locked against relative vertical movement by three depending legs 19 which have lateral extensions 20 and which extend through arcuate slots 22 in the surface 16.

The slots 22 permit the actuator 6 to be rotated on the surface 16 with respect to the base section 4. The rotation can be guided by the slots 22, or by the spigot 17. The parts 4 and 6 can only be disassembled by rotating the actuator 6 so that the lateral extensions 20 are positioned below wide portions 24 at the end of slots 22, which portions are sufficiently wide to allow the extensions 20 to pass therethrough.

The ring 15 of the test structure 8 is connected by three legs 30 to a supporting member 32. The legs 30 are detachable from the supporting member 32, and may be a snap-fit therein. To assemble the device, the legs 30 are separated from the supporting member 32, and the legs then inserted through the cylindrical structure 12 of the base section 4, then through holes 34 in the surface 16, then through arcuate apertures 36 in the reciprocating actuator 6, and are then connected to the supporting member 32.

When assembled, rotation of the actuator 6 with respect to the base section 4 is limited by the engagement of the ends of the apertures 36 with the legs 30 of the test structure, which legs are themselves prevented from rotation by their engagement in the holes 34 in the base section 4, and, in one direction (as in Figures 2 and 4), by the engagement of an operating member 40 on the actuator 6 with an abutment member 42 on the base section 4. Thus, the actuator 6 cannot be rotated sufficiently to allow it to be removed from the base section 4 without first removing the test structure 8.

A spring (not shown) is provided to bias the reciprocating actuator 6 to the position shown in Figures 1 and 3.

The actuator 6 has on its upper side 43 three cams 44 Each cam has an inclined surface 46 and a flat upper surface 48.

The support member 32 is provided on its lower side with three supports 50 having tapered lower ends which, with the actuator in the position shown in Figures 1 and 3, rest on the upper surfaces 48 of the cams 44 to maintain the test structure 8 in its elevated position. When the actuator is moved to the position shown in Figures 2 and 4, the upper surfaces 48 are shifted away from the supports 50 so that the test structure 8 is free to drop. When this has occurred, the actuator 6 is released so that its spring pushes the actuator 6 back to the position shown in Figures 1 and 3, during the course of which the inclined surfaces 46 of the cams 44 engage the supports 50 and thereby raise the test structure back to its elevated position.

The supporting member 32 has two wings 52 and 54 which are disposed on opposite sides to balance each other. The ring 52 is formed with a bore in which a magnet 56 is inserted.

The reciprocating actuator 6 is formed with a bore in which a glass tube 58 is mounted, the end of the tube protruding above the upper surface of the actuator 6. A metal ball 60 is disposed in the tube 58 and normally rests at the lower end of the tube.

In the position of the actuator shown in Figures 1 and 3, the ball 60 and magnet 56 are separated from each other by a relatively large amount, both vertically and laterally. On rotation of the actuator 6 to the position shown in Figures 2 and 4, and before the test structure 8 drops to its low position (i.e. with the structure as shown in phantom in Figure 2), the ball 60 and magnet 56 are no longer separated laterally, but are still vertically separated by an amount sufficient to ensure that the magnet does not shift the ball 60. However, when the test structure 8 drops to its lower position, the attraction of the magnet 56 on the ball 60 is sufficient to cause the ball to shift rapidly to the upper end of the tube 58.

This action is arranged to occur as soon as the test structure 8 has dropped by a predetermined amount, e.g. 1 mm. A sharp, clear "click" is heard, and the ball can be seen through the upper end of the tube 58. Accordingly, there is a clear audible and visual indication that the pour point of the liquid has not yet been reached.

The magnet 56 and ball 60 have to be separated by a fairly large amount to ensure that the ball is no longer attracted and falls to the bottom of the tube 58. This is ensured in the present embodiment by virtue of the fact that when the actuator 6 moves back to the position shown in Figures 1 and 3, the relative movement between the ball 60 and the magnet 56 consists not only of the relatively small vertical movement of the test structure, but also of a relatively large lateral movement caused by the rotation of the actuator 6.

The wing 54 of the supporting member 32 carries a shield member 64 which, with the actuator 6 disposed in the position shown in Figures 1 and 3, blocks an optical path between a lamp and a photosensitive detector (not shown). When the test structure 8 drops after the actuator 6 has been rotated, the shield member 64 moves out of the optical path, so that an electrical signal is provided to indicate that the pour point has not yet been reached.

Preferably, a timer is provided which, after a predetermined interval following the operation of the actuator 6, provides a signal indicating that the pour point has been reached unless it is first disabled by the signal from the photosensitive detector.

It is of course, unnecessary for the device to have both of the indicating arrangements described above, i.e. the magnet 56, tube 58 and ball 60 arrangement, and the shield member 64 and photosensitive detector arrangement. The former of these is preferred if the device is to be used manually, and the latter is preferred for use of the device, possibly with other such devices, in an automated machine for carrying out the testing. In the latter case, it is desirable for the operating member 40 and the shield member 64 of the device to be positioned on substantially the same side so that the machine need only to face that side to operate the device, which facilitates insertion and withdrawal of the device from the machine.

The vessel 10 has a liquid level marking 66 thereon to indicate the level to which the vessel 10 should be filled with liquid. It will be noted that the ring 15 is intended to be fully submerged during testing. This is desired to avoid problems associated with surface tension of the liquid if the ring is to be inserted and withdrawn from the liquid.

For accuracy, it is necessary that the test structure 8 have a predetermined weight. If desired, an additional weight 68 can be attached to the test structure 8, e.g. by snap-fitting onto the top of the supporting member 32.

In use, a thermometer is disposed inside the ring 15, within the liquid. The thermometer extends upwardly, and is held, eg.by ribs 70 on the interior of the central spigot 17 of the base section 4. The temperature-sensitive portion, or bulb, of the thermometer is surrounded by the ring 15 throughout the movement of the ring.

In an alternative to the embodiment described above, the actuator 6 is threadedly mounted on the base section 4 so that it rises and falls as it is rotated. This avoids the need for the cams 44. The thread preferably has a steep pitch to achieve the desired amount of vertical movement of the actuator 6 by a small amount of rotation thereof, as in the above embodiment.

The present invention also extends to apparatus for automatically establishing the temperatures at which liquids cease to flow using a plurality of devices such as those described above. The actuators can be operated by respective operating means which may be of simple design, e.g. solenoids.  A simple timer is provided so that a signal can be provided if the indication means of a device does not provide a signal within a predetermined time limit.  As the temperatures of the liquids are lowered, the respective actuators can be operated simultaneously, in which case only one timer is required, or individually in which case a separate timer can be provided for each device.

1

CLAIMS:

1. A device for establishing the temperature at which a liquid ceases to flow, the device comprising a member (8) movable from a first fixed position under a predetermined force in, or into, the liquid, and indicating means (56,58,60;64) for indicating when the member has reached a second fixed position spaced a predetermined distance from said first fixed position, characterised by a reciprocating actuator (6) which has an initial position in which it supports said member (8) in the member's first fixed position and which is movable away from the initial position to permit the member (8) to move under said force away from the member's first position, the actuator (6) thereafter being movable back to its initial position during the course of which it brings the member back to the member's first position.

2. A device as claimed in claim 1, wherein the actuator (6) is movable between its initial position and a second position, means being provided to bias the actuator (6) into one of its said positions.

3. A device as claimed in claim 2, wherein the actuator (6) is biased to its initial position, the shifting of the actuator (6) to its initial position by the bias also causing the actuator (6) to shift the member (8) back to its first position.

4. A device as claimed in any preceding claim, wherein said actuator (6) supports the weight of the member (8) when the actuator (6) is in its initial position, and allows the member (8) to move under its weight to the member's second position when the actuator (6) is moved away from its initial position.

5. A device as claimed in any preceding claim, wherein the member (8) is allowed to move in or into the liquid by rotating the actuator (6).

6. A device as claimed in claim 5, wherein said actuator (6) is rotated by substantially less than 360°.

7. A device as claimed in any preceding claim, wherein said member (8) and actuator (6) have interengageable portions (50;43,44) to permit the actuator (6) to support the member (8), one of said portions (43,44) having surfaces at at least two levels, whereby the movement of the actuator (6) allows the other portion (50) to engage a different level thereby to permit movement of the member (8).

8.   A device as claimed in claim 7, wherein said one of said portions (43,44) includes a cam (44) which engages the other portion (50) so as to cause the member (8) to be shifted back to its first position upon movement of the actuator (6) back to its initial position.

9.   A device as claimed in any preceding claim, wherein said predetermined distance is not greater than 1 cm.

10.   A device as claimed in claim 9, wherein said predetermined distance is substantially 1 mm.

11.   A device as claimed in any preceding claim, wherein the test member (8) comprises a ring (15) submergeable in the liquid and surrounding a thermometer.

4

12. A device as claimed in any preceding claim, wherein said indicating means (56,58,60) is formed by a part comprising a magnet (56) and a part comprising a member (60) shiftable by the magnet (56) between two positions, one of the parts being mounted on the member (8) so as to cause said shifting to occur when said member (8) has reached its second position.

13. A device as claimed in claim 12, wherein . the other part of said indicating means (56,58,60) is mounted on the actuator (6).

14. A device as claimed in any preceding claim, wherein at least part of said member (8) is made of heat insulating material.

15. A method of establishing the temperature at which a liquid ceases to flow using a device as claimed in any preceding claim, the method comprising repeatedly reciprocating said actuator (6) while lowering the temperature of the liquid, and noting the temperature of the liquid at which, when the actuator (6) is moved away from its initial position, the member (8) fails to move from its first fixed position to its second position within a predetermined time interval.

FIG.1

FIG. 2

FIG.3

FIG.4

FIG.5